# EUROPEAN PATENT SPECIFICATION

(11) **EP 1 608 426 B1**
(45) Date of publication and mention of the grant of the patent: **21.04.2010**
(21) Application number: 04758552.6
(22) Date of filing: 29.03.2004
(51) Int. Cl.: A61F 2/82, A61M 31/00

(54) **IMPLANTABLE MEDICAL DEVICE FOR IN SITU SELECTIVE MODULATION OF AGENT DELIVERY**
IMPLANTIERBARES MEDIZINPRODUKT ZUR SELEKTIVEN MODULATION DER ABGABE VON MITTELN IN SITU
DISPOSITIF MEDICAL IMPLANTABLE POUR LA MODULATION SELECTIVE IN SITU DE L'ADMINISTRATION D'UN AGENT

(30) Priority: 28.03.2003 US 458906 P; 26.03.2004 US 810241
(43) Date of publication of application: 28.12.2005
(60) Divisional application: 07023181.6
(73) Proprietor: Conor Medsystems, Inc., Menlo Park, CA 94025 (US)
(72) Inventor: SHANLEY, John, F., Redwood City, CA 94061 (US); PARKER, Theodore, J., Danville, CA 94506 (US); EIGLER, Neal, L., Pacific Palisades, CA 90272 (US)
(74) Representative: Schorr, Frank Jürgen
(86) International application number: PCT/US2004/009604
(87) International publication number: WO 2004/087251

(56) References cited:
- EP-A- 1 277 449
- WO-A-03/015664
- US-A- 4 989 601
- US-A1- 2002 082 679
- US-A1- 2002 128 704

## Description

### Field of the Invention

The invention relates, to an implantable medical device and method for delivery of an agent to a location within a patient, and more particularly, the invention relates to the selective modulation of agent delivery from the implantable medical device.

### Description of the Related Art

Implantable medical devices are often used for delivery of a beneficial agent, such as a drug, to an organ or tissue in the body at a controlled delivery rate over an extended period of time. These devices may deliver agents to a wide variety of bodily systems to provide a wide variety of treatments.

One of the many implantable medical devices which have been used for local delivery of beneficial agents is the vascular stent, Vascular stents are typically introduced percutaneously, and transported transluminally until positioned at a desired location. These devices are then expanded either mechanically, such as by the expansion of a mandrel or balloon positioned inside the device, or expand themselves by releasing stored energy upon actuation within the body. Once expanded within the lumen, these devices, called stents, become encapsulated within the body tissue and remain a permanent implant.

Known stent designs include monofilament wire coil stents (U.S. Pat. No. 4,969,458); welded metal cages (U.S. Pat. Nos. 4;733,665 and 4,776,337); and, most prominently, thin-walled metal cylinders with axial slots formed around the circumference (U.S. Pat. Nos. 4,733,665; 4,739,762; and 4,776,337). Know construction materials for use in stents include polymers, organic fabrics and biocompatible metals, such as stainless steel, gold, silver, tantalum, titanium, cobalt based alloys, and shape memory alloys, such as Nitinol.

Of the many problems that may be addressed through stent-based local delivery of beneficial agents, one of the most important is restenosis. Restenosis is a major complication that can arise following vascular interventions such as angioplasty and the implantation of stents. Simply defined, restenosis is a wound healing process that reduces the vessel lumen diameter by extracellular matrix deposition, neointimal hyperplasia, and vascular smooth muscle cell proliferation, and which may ultimately result in renarrowing or even reocclusion of the lumen. Despite the introduction of improved surgical techniques, devices, and pharmaceutical agents, the overall restenosis rate is still reported in the range of 25% to 50% within six to twelve months after an angioplasty procedure. To treat this condition, additional revascularization procedures are frequently required, thereby increasing trauma and risk to the patient.

One of the techniques under development to address the problem of restenosis is the use of various beneficial agents in or on stents. U.S. Pat. No. 5,716,981, for example, discloses a stent that is surface-coated with a composition comprising a polymer carrier and paclitaxel (a well-known compound that is commonly used in the treatment of cancerous tumors). The patent offers detailed descriptions of methods for coating stent surfaces, such as spraying and dipping, as well as the desired character of the coating itself: it should "coat the stent smoothly and evenly" and "provide a uniform, predictable, prolonged release of the anti-angiogenic factor." Surface coatings, however, can provide little actual control over the release kinetics of beneficial agents. These coatings are necessarily very thin, typically 5 to 8 microns deep. The surface area of the stent, by comparison is very large, so that the entire volume of the beneficial agent has a very short diffusion path to discharge into the surrounding tissue.

Increasing the thickness of the surface coating has the beneficial effects of improving drug release kinetics including the ability to control drug release and to allow increased drug loading. However, the increased coating thickness results in increased overall thickness of the stent wall. This is undesirable for a number of reasons, including increased trauma to the vessel wall during implantation, reduced flow cross-section of the lumen after implantation, and increased vulnerability of the coating to mechanical failure or damage during expansion and implantation. Coating thickness is one of several factors that affect the release kinetics of the beneficial agent, and limitations on thickness thereby limit the range of release rates, duration of drug delivery, and the like that can be achieved.

In addition to sub-optimal release profiles, there are further problems with surface coated stents. The fixed matrix polymer carriers frequently used in the device coatings typically retain approximately 30% - 80% of the beneficial agent in the coating indefinitely. Since these beneficial agents are frequently highly cytotoxic, sub-acute and chronic problems such as chronic inflammation, late thrombosis, and late or incomplete healing of the vessel wall may occur. Additionally, the carrier polymers themselves are often highly inflammatory to the tissue of the vessel wall. On the other hand, use of biodegradable polymer carriers on stent surfaces can result in the creation of "virtual spaces" or voids between the stent and tissue of the vessel wall after the polymer carrier has degraded, which permits differential motion between the stent and adjacent tissue. Resulting problems include micro-abrasion and inflammation, stent drift, and failure to re-endothelialize the vessel wall.

One drawback of known stents with drugs in or on the stents is the inability to tailor drug delivery to the tissue structure present adjacent the implanted device. The drug delivered from a drug delivery stent is delivered to all the tissue supported by the stent including both the diseased tissue (lesion) and neighboring relatively healthy tissue. The delivery of an anti-restenosis drug or other drugs to relatively healthy tissue in addition to the tissue to be treated may in some cases cause damage to this relatively healthy tissue. For example, aneurysms have been observed to form in the wall of a blood vessel in a portion of the blood vessel which is supported by the stent and has no apparent lesions, i.e. the relatively healthy tissue close to the diseased tissue, while similar anueysms have not been observed in the diseased tissue. However, due to a combination of stent placement inaccuracies and a requirement to support the entire diseased tissue site, a stent must support both the diseased tissue and some relatively healthy tissue. It may also be desirable to deliver variable amounts of the drug to different tissue for many other reasons.

Patent application US 2002/0128704 A1 discloses a method for controlling the activity of drugs on or in drug-coated or drug-loaded implantable metallic devices. The method uses inductive heating of such devices. The heating of the device is used to activate drugs that have little activity at body temperature, to enhance for defined periods the reactive environment at the stent for drug-adjacent tissue interactions, or to deactivate drugs upon heating, employing reverse effects.

European patent application EP 1 277 449 A1 discloses an implantable medical device including a beneficial agent arranged in recesses for delivery to an implantation site within a patient. The recesses are distributed on the surface of the stent.

International application WO 03/015664 A1 discloses an expandable medical device in which a beneficial agent is loaded into openings in the struts of the device. The agent is configured to achieve a desired delivery profile.

Accordingly, it would be desirable to provide an implantable medical device for delivery of agents, such as drugs, to a patient and selectively modulating, activating, or deactivating agent delivery after implantation to provide agents at targeted tissue areas adjacent the expandable medical device.

### Summary of the Invention

The present invention provides a stent.

In a device aspect, the present invention provides an implantable medical device according to claim 1.

### Brief Description of the Drawing Figures

The invention will now be described in greater detail with reference to the preferred embodiments illustrated in the accompanying drawings, in which like elements bear like reference numerals, and wherein:

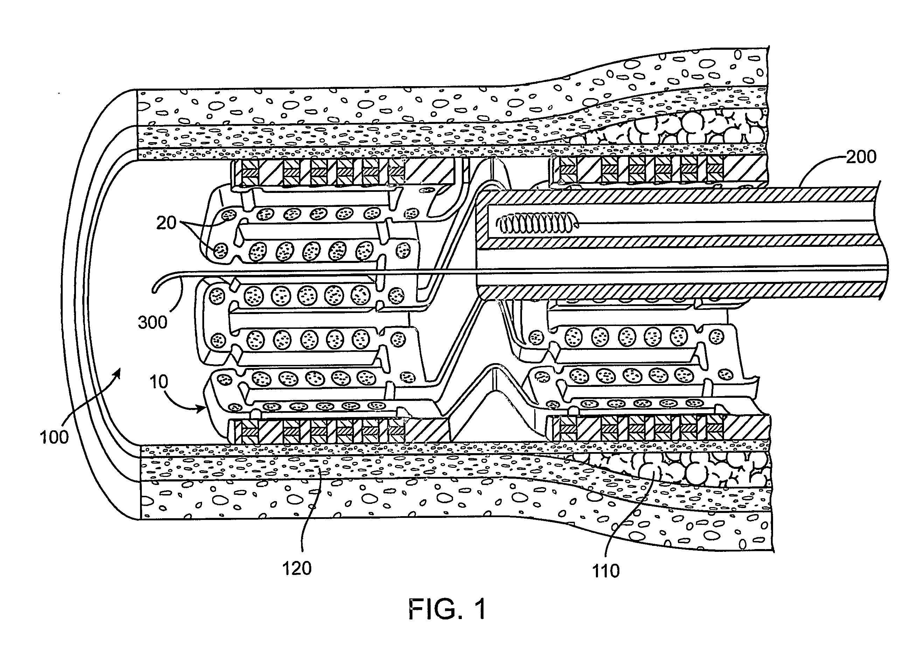

FIG. 1 is a side cross sectional view of an implantable beneficial agent delivery device and activation/deactivation means according to one example of the invention.

### Detailed Description of the Invention

The device of the invention comprises a stent and a drug delivery material therein or thereon such that the amount of drug delivered from one region of the stent can be modulated relative to the amount delivered from another region of the stent, such differential modulation being specified after the deployment of the stent into a mammal.

FIG. 1 illustrates an implantable medical device 10 implanted within a blood vessel 100. As shown in FIG. 1, the blood vessel 100 includes a lesion 110 which has been expanded by expansion of the medical device 10. In addition to supporting the lesion 110 or other unhealthy tissue, the expanded medical device 10 also supports one or more regions of relatively healthy blood vessel tissue 120. In the illustrated example, the relatively healthy tissue 120 is adjacent an end region of the medical device 10.

The expandable medical device 10 includes a plurality of openings 20 containing a beneficial agent to be delivered to the tissue. Some of the openings 20 are adjacent the lesion 110 and some of the openings are adjacent to the relatively healthy tissue 120. An activating/deactivating device can be in the form of a catheter 200 which is configured to be delivered over a guidewire 300 into the lumen of the medical device 10 after implantation to activate and/or deactivate the beneficial agent(s) within the openings 20 to provide targeted modulation of delivery of the beneficial agent to deliver the beneficial agent to specific regions or locations from a surface of the implanted medical device 10.

In the case of a beneficial agent which is useful for reducing extracellular matrix deposition, neointimal hyperplasia, and vascular smooth muscle cell proliferation, which may ultimately result in renarrowing or even reocclusion of the lumen the beneficial agent can be activated adjacent the lesion 110 or deactivated adjacent to relatively healthy tissue 120 to deliver the majority of the beneficial agent, preferably a therapeutically effective amount of the beneficial agent, to the targeted tissue.

The term "modulation" refers to the adjustment of the amount of beneficial agent delivered or the beneficial agent delivery rate either by activating or deactivating. Modulation of the amount of drug delivered is envisioned to include the range from no delivery of drug contained in a specified region, to delivery of a portion of the drug from a region, to delivery of the entire drug contained in a specified region of the stent.

The terms "activating and deactivating" refer to the activation or release of a beneficial agent or the deactivation, blocking, or removal of a beneficial agent. It may include altering the properties of the beneficial agent to render it active or inactive. It may also include altering materials surrounding the beneficial agent to increase release or decrease or prevent release of the beneficial agent. It may further include degrading or removing a compound in a barrier or matrix layer to allow delivery of a beneficial agent or to allow removal of a beneficial agent, such as by flushing the agent away in the blood stream. Activation generally results in delivery of a therapeutic agent of a therapeutically effective amount at a therapeutically effective release rate. Deactivation generally results in delivery of a therapeutically ineffective amount or delivery at a therapeutically ineffective release rate. Activation generally results in increased rates of delivery of an agent to target tissue as compared to the delivery rate prior to activation, while deactivation generally results in decreased rates of delivery of an agent to the target tissue as compared to the delivery rate prior to deactivation.

The term "beneficial agent" as used herein is intended to have the broadest possible interpretation and is used to include any therapeutic agent or drug, as well as inactive agents such as barrier layers, carrier layers, therapeutic layers or protective layers. The beneficial agent may be comprised of a drug alone, or may additionally contain nondrug material to act as a matrix or binder to hold the drug containing material within or on the stent and / or modulate the release of the drug from a region of the stent.

The terms "drug" and "therapeutic agent" are used interchangeably to refer to any therapeutically active substance that is delivered to tissue of a living being to produce a desired, usually beneficial, effect. The present invention is particularly well suited for the delivery of antineoplastic, angiogenic factors, immuno-suppressants, and antiproliferatives (anti-restenosis agents) such as paclitaxel and Rapamycin for example, and antithrombins.

The term "matrix" or "biocompatible matrix" are used interchangeably to refer to a medium or material that, upon implantation in a subject, does not elicit a detrimental response sufficient to result in the rejection of the matrix. The matrix typically does not provide any therapeutic responses itself, though the matrix may contain or surround a therapeutic agent, a therapeutic agent, an activating agent or a deactivating agent, as defined herein. A matrix is also a medium that may simply provide support, structural integrity or structural barriers. The matrix may be polymeric, non-polymeric, hydrophobic, hydrophilic, lipophilic, amphiphilic, and the like.

The term "bioerodible" refers to a matrix, as defined herein, that is bioresorbable and/or can be broken down by either chemical or physical process, upon interaction with a physiological enviromnent. The bioerodible matrix is broken into components that are metabolizable or excretable, over a period of time from minutes to years, preferably less than one year, while maintaining any requisite structural integrity in that same time period.

Although the invention is described herein with reference to the example of a stent containing a beneficial agent within holes for delivery of the beneficial agent to the walls of a lumen, it should be understood that other devices may be used and the devices may be used to treat other tissue structures. For example, the drug delivery device can be an expandable vascular stent, urethral stent, biliary stent, or shunt. The beneficial agent from the implantable medical device can be differentially delivered to any two different types of tissues or tissue structures, for example, different tissue or tissue structures may include plaques of different types, healthy tissue, cancerous tissues, injured tissue, and tissue adjacent various structures, such as bifurcations.

The term "polymer" refers to molecules formed from the chemical union of two or more repeating units, called monomers. Accordingly, included within the term "polymer" may be, for example, dimers, trimers and oligomers. The polymer may be synthetic, naturally-occurring or semisynthetic. In preferred form, the term "polymer" refers to molecules which typically have a M_{w} greater than about 3000 and preferably greater than about 10,000 and a M_{w} that is less than about 10 million, preferably less than about a million and more preferably less than about 200,000. Examples of polymers include but are not limited to, poly-α-hydroxy acid esters such as, polylactic acid (PLLA or DLPLA), polyglycolic acid, polylactic-co-glycolic acid (PLGA), polylactic acid-co-caprolactone; poly (block-ethylene oxide-block-lactide-co-glycolide) polymers (PEO-block-PLGA and PEO-block-PLGA-block-PEO); polyethylene glycol and polyethylene oxide, poly (block-ethylene oxide-block-propylene oxide-block-ethylene oxide); polyvinyl pyrrolidone; polyorthoesters; polysaccharides and polysaccharide derivatives such as polyhyaluronic acid, poly (glucose), polyalginic acid, chitin, chitosan, chitosan derivatives, cellulose, methyl cellulose, hydroxyethylcellulose, hydroxypropylcellulose, carboxymethylcellulose, cyclodextrins and substituted cyclodextrins, such as beta-cyclodextrin sulfobutyl ethers; polypeptides and proteins, such as polylysine, polyglutamic acid, albumin; polyanhydrides; polyhydroxy alkonoates such as polyhydroxy valerate, polyhydroxy butyrate, and the like.

It is envisioned that the pattern of regions on the stent created which deliver differing amounts of the drug contained in each region be specified based on the physiology and pathology of a region of a lumen or other tissue being treated by the medical device and drug or therapeutic agent. Further, the regions are envisioned to include both regions on the mural side of the stent and on the luminal side of the stent.

It is envisioned in one embodiment that the stent will include beneficial agent that is initially disposed in a spatially uniform manner in the stent and later specific areas of the stent are treated differentially by an activating/deactivating means to create a regio-specific pattern of amount of agent to be delivered after deployment of the stent. Additionally, the beneficial agent may initially contain a pro-drug or a blocked drug which by later treatment is converted from a therapeutically inactive form into the active drug for delivery from that specific area.
This process is generally called activation of a drug.

Conversely, starting with a stent with a uniform distribution of beneficial agent therein, it is envisioned to create an inhomogeneous pattern of regions that will release active drug and regions that will not release or will only release a limited amount of active drug by selectively degrading or otherwise rendering the drug contained in specific regions of the stent therapeutically inactive. This process is generally called deactivation of a drug.

Further, it is envisioned to render the drug containing material in a specific region more able or less able to release the drug contained in that region by selectively treating and effecting a modification the non-drug component of the drug delivery material to provide a modulation of drug release amount. This process is generally called activation or deactivation by modification of the matrix.

It is also envisioned to render the beneficial agent in a specific region more or less able to be released in that region by selectively treating and modifying a structure other than the beneficial agent. For example, a barrier layer may be made more or less permeable to the drug to allow the drug to be delivered, to substantially prevent drug delivery, or to allow the drug to be delivered and washed away by the blood stream or other means.

Various activation/deactivation means including energy and chemicals are envisioned to effect the modulation of drug delivery capability of specific drug containing regions on the stent by modification variously of the drug or a pro-drug, or of the matrix or binder component of the drug delivery material, or of another structure, such as a barrier layer.

In the embodiment illustrated in FIG. 1, the openings 20 in the stent 10 allow easy accessability for the activating/deactivating means in the form of chemicals to be delivered to the beneficial agent. Without the openings, it may be difficult for some chemicals to be delivered from an activation/deactivation means within the lumen of the stent to a mural side of the stent to perform the activation or deactivation.

It is envisioned that a drug in a specific region of the medical device that is not in an active form in the initial beneficial agent is activated by treating the drug to change the ambient pH value, or by degrading an antagonist to the drug, or by adding an activating co-factor such as a metal salt or an enzyme that will convert the drug to an active form. The drug may initially be present in a blocked form and may be activated by treatment with a de-blocking agent such as an acid, base, or salt. The drug may initially be present in the beneficial agent as a pro-drug where the drug is bound to another moiety or polymer via a labile linkage, in which case it cannot be released as an active drug, but by treatment variously with an enzymatic agent, such as if the labile linkage contains peptide bonds.

Conversely, it is envisioned that in a system where the beneficial agent is initially present so that the distribution of drug is uniform along the stent, a pattern of drug releasing and non-drug releasing regions may be created by degrading or otherwise rendering the drug in some regions therapeutically inactive by local treatment of specific regions on the stent. A chemical agent may be added to bind to the drug to inactivate it, or an oxidant or reductant added to chemically modify the therapeutic agent. The species effecting the chemical de-activation of the drug in a local region may be generated in situ, such as by the action of actinic radiation to create oxidizing species.

A chemical agent to degrade a stabilizer for the drug contained in the drug delivery material is envisioned as a method to decrease or eliminate the ability to deliver active drug from that region.

Various treatments applied to the matrix or binder component of the drug delivery material are envisioned as methods to locally control the delivery of drug from a region of the stent. In one embodiment, treatment of a polymer matrix that contains reactive groups capable of cross-linking the matrix and holding the drug more tenaciously with a cross-linking reagent is envisioned as a method to decrease the amount of drug that can be delivered from a region so treated. Conversely, a polymer matrix containing linkages that are chemically labile can result in a matrix that is unable to hold drug effectively and drug in these regions will be released prematurely and create regions depleted of drug in the area for therapeutic treatment.

It is envisioned that the drug delivery material may be disposed on the surfaces of the stent in various configurations, including within volumes defined by the stent, such as holes or concave surfaces, as a reservoir of drug, as a coating on all or a portion of surfaces of the stent structure, within the device material itself, as a sleeve of material positioned over the device, as agent threads woven through the device, or in any other configuration.

When the drug delivery material is disposed within holes in the strut structure of the stent to form a reservoir, the holes may be partially or completely filled with material. Additionally, the composition of the drug delivery material within the holes may be comprised of a plurality of individual layers, each with the same or different compositions with respect to the amount of drug and the amount of matrix or binding material comprising the drug delivery material. It is further envisioned that in the above described activation or deactivation of material located at specific sites or regions of the stent, drug containing material may comprise one drug in one region of the stent and another drug in a region distinct from the first region, such that two or more drugs may be independently treated to provide independent region specific patterns for simultaneous or sequential delivery of two or more drugs or therapeutic agents. More than one beneficial agent can be provided in alternating or interspersed holes for a uniform initial arrangement of more than one beneficial agent across the stent surface, wherein one agent is delivered without activation and one agent requires activation. Alternately, more than one beneficial agent may be activated or deactivated simultaneously or sequentially by the same or different activation/deactivation means.

Barrier layers can also be formulated to be activated by an agent which could change the porosity of the barrier layer and/or change the rate of biodegradation of the barrier layer or the bulk beneficial agent carrier. In each case, release of the beneficial agent could be activated by the physician at will by delivery of the agent.

Further, devices capable of effecting the above described region-specific drug activating or deactivating treatment after deployment of the stent are envisioned. Such activating/deactivating devices may be positioned either inside or outside the body. According to one example, the device is a catheter-based device capable of percutaneous transluminal placement to position an activating/deactivating portion of the catheter within the lumen of the stent such that activating or deactivating treatment of the beneficial agent occurs from the luminal surface of the stent by operation of a distal area of the activating/deactivating device.

The catheter-based treatment device is preferably translated to the deployed drug delivery stent in the same manner as the balloon tipped catheter used to deploy the drug delivery stent. It is envisioned that the design of the device to effect activation or deactivation of the drug in the beneficial agent will be governed by the nature of the treatment.

One example of an activation/deactivation catheter using a chemical or biological activation/deactivation means includes balloon catheter which is impregnated or coated with the activation/deactivation agent. The activation/deactivation agent can be released from the balloon by the selective application of energy from the interior of the inflated balloon in one of the manners described above. In operation, the activation/deactivation balloon catheter is inserted into the stent and inflated so that the chemical agent, in a therapeutically inactive form, is present on the balloon directly adjacent the interior surface of the stent. The chemical agent can be chemically inactive or sequestered by a matrix, barrier, or other material until released. The chemical agent is then activated in selected region(s), such as by application of ultrasonic or other energy from a controllable energy source within the balloon. The chemical agent released from the balloon acts on the beneficial agent, drug, barrier layer, matrix, or binder to activate/deactivate drug delivery in any one of the manners described above.

The use of a balloon catheter based activation/deactivation means has the additional advantage of eliminating blood from the site during activation/deactivation which can improve visibility and accuracy.

For the delivery of chemical agents to activate or deactivate drug in drug containing layers, a catheter with a porous balloon on its distal end where the individual pores or areas of pores of the balloon are connected to individual tubes whose delivery of agent can be controlled is envisioned. Additionally, a balloon is envisioned whose surface is porous is some areas and non-porous in others in a pattern to match the desired pattern of drug activation and deactivation on the stent.

The methods using the present invention generally include implanting an implantable medical device having a beneficial agent with known procedures. For example, a stent may be placed and expanded with a balloon catheter which is delivered transluminaly over a guidewire under fluoroscopic visualization. Upon implantation of the medical device, an activation/deactivation means is used to selectively activate/deactivate drug delivery in selected regions of the implanted device. For example, an activation/deactivation device on the end of a catheter can be inserted over the guidewire to the location of an implanted stent and the beneficial agent of the stent can be activated/deactivated in regions by the surgeon by "painting" with the catheter by delivery of chemicals under fluoroscopic visualization.

Alternatively, the step of activating/deactivating may be performed by a activating/deactivating catheter which is maintained fixed and is activated in specific regions from an exterior of the body. For example, a balloon catheter including as plurality of individual activatable electric circuits, fiber optics, or chemical delivery ports can deliver the activating/deactivating chemical according to a pattern selected by a sturgeon by drawing on a computer image of the tissue surrounding the implant.

As described herein, the beneficial agent is preferably provided in the implantable medical device in reservoirs in a solid or non flowable form, such as in a polymer matrix or gel. The activation/deactivation means is capable of activating or deactivating the agent in the reservoirs without the need for circuitry or power sources in the implantable device.

In another example, the agent delivery from substantially the entire stent or other device may be activated or deactivated after implantation to begin or terminate a treatment determined to be necessary after implantation. For example, a stent containing an anti-restenotic agent and a second agent, such as an angiogenic agent, may be delivered with the stent initially releasing primarily the anti-restenotic agent. At a later date, the delivery of the second agent can be initiated by an activating means when it is determined that the condition of the patient requires the second agent. For example, the angiogenic agent may be released upon a determination that the heart tissue has reached an undesirable level of ischemia at which a second agent would be beneficial. The second agent may treat a variety of conditions, for example, the second agent may include a vasodilator, an angiogenic agent or combination of angiogenic agents, insulin, or the like. The activation can be achieved by any of the means described above. In addition, chemical activation by a systemically applied agent may be used to activate a second drug on substantially the entire stent.

In an example, the agent delivered from substantially the entire stent or other device can be deactivated upon determination by the physician that the patient's condition no longer requires the delivery of the drug. This deactivation can be done in any of the manners described above or by systemic administration of a chemical deactivation means.

Therapeutic agents for use with the present invention may, for example, take the form of small molecules, peptides, lipoproteins, polypeptides, polynucleotides encoding polypeptides, lipids, protein-drugs, protein conjugate drugs, enzymes, oligonucleotides and their derivatives, ribozymes, other genetic material, cells, antisense oligonucleotides, monoclonal antibodies, platelets, prions, viruses, bacteria, eukaryotic cells such as endothelial cells, stem cells, ACE inhibitors, monocyte/macrophages and vascular smooth muscle cells. Such agents can be used alone or in various combinations with one another. For instance, anti-inflammatories may be used in combination with antiproliferatives to mitigate the reaction of tissue to the antiproliferative. The therapeutic agent may also be a pro-drug, which metabolizes into the desired drug when administered to a host. In addition, therapeutic agents may be pre-formulated as microcapsules, microspheres, microbubbles, liposomes, niosomes, emulsions, dispersions or the like before they are incorporated into the matrix. Therapeutic agents may also be radioactive isotopes or agents activated by some other form of energy such as light or ultrasonic energy, or by other circulating molecules that can be systemically administered.

Exemplary classes of therapeutic agents include antiproliferatives, antithrombins (i.e., thrombolytics), immunosuppressants, antilipid agents, anti-inflammatory agents, antineoplastics including antimetabolites, antiplatelets, angiogenic agents, anti-angiogenic agents, vitamins, antimitotics, metalloproteinase inhibitors, NO donors, nitric oxide release stimulators, anti-sclerosing agents, vasoactive agents, endothelial growth factors, beta blockers, hormones, statins, insulin growth factors, antioxidants, membrane stabilizing agents, calcium antagonists (i.e., calcium channel antagonists), retinoids, anti-macrophage substances, antilymphocytes, cyclooxygenase inhibitors, immunomodulatory agents, angiotensin converting enzyme (ACE) inhibitors, anti-leukocytes, high-density lipoproteins (HDL) and derivatives, cell sensitizers to insulin, prostaglandins and derivatives, anti-TNF compounds, hypertension drugs, protein kinases, antisense oligonucleotides, cardio protectants, petidose inhibitors (increase blycolitic metabolism), endothelin receptor agonists, interleukin-6 antagonists, anti-restenotics, and other miscellaneous compounds.

Antiproliferatives include, without limitation, sirolimus, paclitaxel, actinomycin D, rapamycin, and cyclosporin.

Antithrombins include, without limitation, heparin, plasminogen, α₂-antiplasmin, streptokinase, bivalirudin, and tissue plasminogen activator (t-PA).

Immunosuppressants include, without limitation, cyclosporine, rapamycin and tacrolimus (FK-506), sirolumus, everolimus, etoposide, and mitoxantrone.

Antilipid agents include, without limitation, HMG CoA reductase inhibitors, nicotinic acid, probucol, and fibric acid derivatives (e.g., clofibrate, gemfibrozil, gemfibrozil, fenofibrate, ciprofibrate, and bezafibrate).

Anti-inflammatory agents include, without limitation, salicylic acid derivatives (e.g., aspirin, insulin, sodium salicylate, choline magnesium trisalicylate, salsalate, dflunisal, salicylsalicylic acid, sulfasalazine, and olsalazine), para-amino phenol derivatives (e.g., acetaminophen), indole and indene acetic acids (e.g., indomethacin, sulindac, and etodolac), heteroaryl acetic acids (e.g., tolmetin, diclofenac, and ketorolac), arylpropionic acids (e.g., ibuprofen, naproxen, flurbiprofen, ketoprofen, fenoprofen, and oxaprozin), anthranilic acids (e.g., mefenamic acid and meclofenamic acid), enolic acids (e.g., piroxicam, tenoxicam, phenylbutazone and oxyphenthatrazone), alkanones (e.g., nabumetone), glucocorticoids (e.g., dexamethaxone, prednisolone, and triamcinolone), pirfenidone, and tranilast.

Antineoplastics include, without limitation, nitrogen mustards (e.g., mechlorethamine, cyclophosphamide, ifosfamide, melphalan, and chlorambucil), methylnitrosoureas (e.g., streptozocin), 2-chloroethylnitrosoureas (e.g., carmustine, lomustine, semustine, and chlorozotocin), alkanesulfonic acids (e.g., busulfan), ethylenimines and methylmelamines (e.g., triethylenemelamine, thiotepa and altretamine), triazines (e.g., dacarbazine), folic acid analogs (e.g., methotrexate), pyrimidine analogs (5-fluorouracil, 5-fluorodeoxyuridine, 5-fluorodeoxyuridine monophosphate, cytosine arabinoside, 5-azacytidine, and 2',2'-difluorodeoxycytidine), purine analogs (e.g., mercaptfor use with the present invention may, for example, take the form of small molecules, peptides, lipoproteins, polypeptides, polynucleotides encoding polypeptides, lipids, protein-drugs, protein conjugate drugs, enzymes, oligonucleotides and their derivatirubicin, idarubicin, epirubicin, mitoxantrone, bleomycins, plicamycin and mitomycin), phenoxodiol, etoposide, and platinum coordination complexes (e.g., cisplatin and carboplatin).

Antiplatelets include, without limitation, insulin, dipyridamole, tirofiban, eptifibatide, abciximab, and ticlopidine.

Angiogenic agents include, without limitation, phospholipids, ceramides, cerebrosides, neutral lipids, triglycerides, diglycerides, monoglycerides lecithin, sphingosides, angiotensin fragments, nicotine, pyruvate thiolesters, glycerol-pyruvate esters, dihydoxyacetone-pyruvate esters and monobutyrin.

Anti-angiogenic agents include, without limitation, endostatin, angiostatin, fumagillin and ovalicin.

Vitamins include, without limitation, water-soluble vitamins (e.g., thiamin, nicotinic acid, pyridoxine, and ascorbic acid) and fat-soluble vitamins (e.g., retinal, retinoic acid, retinaldehyde, phytonadione, menaqinone, menadione, and alpha tocopherol).

Antimitotics include, without limitation, vinblastine, vincristine, vindesine, vinorelbine, paclitaxel, docetaxel, epipodophyllotoxins, dactinomycin, daunorubicin, doxorubicin, idarubicin, epirubicin, mitoxantrone, bleomycins, plicamycin and mitomycin.

Metalloproteinase inhibitors include, without limitation, TIMP-1, TIMP-2, TIMP-3, and SmaPI.

NO donors include, without limitation, L-arginine, amyl nitrite, glyceryl trinitrate, sodium nitroprusside, molsidomine, diazeniumdiolates, S-nitrosothiols, and mesoionic oxatriazole derivatives.

NO release stimulators include, without limitation, adenosine.

Anti-sclerosing agents include, without limitation, collagenases and halofuginone.

Vasoactive agents include, without limitation, nitric oxide, adenosine, nitroglycerine, sodium nitroprusside, hydralazine, phentolamine, methoxamine, metaraminol, ephedrine, trapadil, dipyridamole, vasoactive intestinal polypeptides (VIP), arginine, and vasopressin.

Endothelial growth factors include, without limitation, VEGF (Vascular Endothelial Growth Factor) including VEGF-121 and VEG-165, FGF (Fibroblast Growth Factor) including FGF-1 and FGF-2, HGF (Hepatocyte Growth Factor), and Ang1 (Angiopoietin 1).

Beta blockers include, without limitation, propranolol, nadolol, timolol, pindolol, labetalol, metoprolol, atenolol, esmolol, and acebutolol.

Hormones include, without limitation, progestin, insulin, the estrogens and estradiols (e.g., estradiol, estradiol valerate, estradiol cypionate, ethinyl estradiol, mestranol, quinestrol, estrond, estrone sulfate, and equilin).

Statins include, without limitation, mevastatin, lovastatin, simvastatin, pravastatin, atorvastatin, and fluvastatin.

Insulin growth factors include, without limitation, IGF-1 and IGF-2.

Antioxidants include, without limitation, vitamin A, carotenoids and vitamin E.

Membrane stabilizing agents include, without limitation, certain beta blockers such as propranolol, acebutolol, labetalol, oxprenolol, pindolol and alprenolol.

Calcium antagonists include, without limitation, amlodipine, bepridil, diltiazem, felodipine, isradipine, nicardipine, nifedipine, nimodipine and verapamil.

Retinoids include, without limitation, all-trans-retinol, all-trans-14-hydroxyretroretinol, all-trans-retinaldehyde, all-trans-retinoic acid, all-trans-3,4-didehydroretinoic acid, 9-cis-retinoic acid, 11-cis-retinal, 13-cis-retinal, and 13-cis-retinoic acid.

Anti-macrophage substances include, without limitation, NO donors.

Anti-leukocytes include, without limitation, 2-CdA, IL-1 inhibitors, anti-CD116/CD18 monoclonal antibodies, monoclonal antibodies to VCAM, monoclonal antibodies to ICAM, and zinc protoporphyrin.

Cyclooxygenase inhibitors include, without limitation, Cox-1 inhibitors and Cox-2 inhibitors (e.g., CELEBREX® and VIOXX®).

Immunomodulatory agents include, without limitation, immunosuppressants (see above) and immunostimulants (e.g., levamisole, isoprinosine, Interferon alpha, and Interleukin-2).

ACE inhibitors include, without limitation, benazepril, captopril, enalapril, fosinopril sodium, lisinopril, quinapril, ramipril, and spirapril.

Cell sensitizers to insulin include, without limitation, glitazones, P par agonists and metformin.

Antisense oligonucleotides include, without limitation, resten-NG.

Cardio protectants include, without limitation, VIP, pituitary adenylate cyclase-activating peptide (PACAP), apoA-I milano, amlodipine, nicorandil, cilostaxone, and thienopyridine.

Petidose inhibitors include, without, limitation, omnipatrilat.

Anti-restenotics include, without limitation, include vincristine, vinblastine, actinomycin, epothilone, paclitaxel, and paclitaxel derivatives (e.g., docetaxel).

Miscellaneous compounds include, without limitation, Adiponectin.

While the invention has been described in detail with reference to the preferred embodiments thereof, it will be apparent to one skilled in the art that various changes and modifications can be made and equivalents employed, without departing from the present invention, as defined in the appended claims.

## Claims

1. A stent (10) comprising:
a device body including a beneficial agent comprising a drug arranged for delivery from the device body to an implantation site within a patient, the beneficial agent configured to be selectively modulated after implantation within the patient by an activating/deactivating means (200),
wherein the beneficial agent at a first region of the device body can be modulated by the activating/ deactivating means to create a different agent delivery profile than the beneficial agent at a second region of the device body,
**characterized in that**
the beneficial agent is configured to be modulated after implantation within the patient by the activating/deactivating means (200) in the form of a chemical agent,
the amount of drug delivered in use from a first region of the device (10) can be modulated by the activating/ deactivating means (200) without modulating the amount of beneficial agent delivered from a second region of the device (10).

2. The device of Claim 1, wherein the beneficial agent is substantially uniformly distributed in the device body.

3. The device of Claim 1, comprising a barrier layer configured to be acted on by the activating/deactivating means (200) to release or retain the beneficial agent in the first or second regions.

4. The device of Claim 1, wherein beneficial agent is contained in a matrix or binder configured to be acted on by the activating/deactivating means (200) to release or retain the beneficial agent in the first or second regions.

5. The device of Claim 1, wherein the device body is a cylindrical, expandable medical device (10).

6. The device of Claim 5, wherein the device is a stent.

7. The device of Claim 5, wherein the beneficial agent is contained in a plurality of recesses (20) in the medical device (10).

8. The device of Claim 5, wherein the beneficial agent is contained in a plurality of through holes (20) in the medical device (10).

9. The device of Claim 1, wherein the beneficial agent is selected to treat vascular disease.

10. The device of Claim 1, wherein the beneficial agent is located in a plurality of openings (20) in the device body (10).

11. The stent according to claim 1, being an expandable implantable stent (10), wherein the beneficial agent is affixed to the stent (10), the beneficial agent having an initial agent release profile; further comprising the activating/deactivating means (200) to form a beneficial agent delivery system, wherein the beneficial agent release profile can be modulated by the activating/ deactivating means (200) after implantation of the stent (10) within the patient to create an agent release profile different from the initial agent release profile.

12. The system of Claim 11, wherein the beneficial agent is substantially uniformly distributed in the device body (10).

13. The system of Claim 11, comprising a barrier layer configured to be acted on by the activating/deactivating means (200) to create the different agent release profile.

14. The system of Claim 11, wherein beneficial agent is contained in a matrix or binder configured to be acted on by the activating/deactivating means (200) to release or retain the beneficial agent in the first or second regions.

15. The system of Claim 11, wherein the beneficial agent is contained in a plurality of recesses (20) in the stent (10).

16. The system of Claim 11, wherein the beneficial agent is contained in a plurality of through holes (20) in the stent (10).

17. The system of Claim 11, wherein the beneficial agent is selected to treat vascular disease.

18. The system of Claim 11, wherein the beneficial agent is configured for substantially no release until modulation by the activating/deactivating means (200).

19. The system of Claim 11, further comprising an additional beneficial agent affixed to the stent (10) and configured to be released without activation.

## Patentansprüche

1. Stent (10), der Folgendes umfasst:
einen Vorrichtungskörper, der ein nützliches Agens beinhaltet, das ein Arzneimittel umfasst, angeordnet zur Abgabe aus dem Vorrichtungskörper an eine Implantationsstelle innerhalb eines Patienten, wobei das nützliche Agens dafür konfiguriert ist, nach der Implantation innerhalb des Patienten durch ein Aktivierungs-/Deaktivierungsmittel (200) selektiv moduliert zu werden,
wobei das nützliche Agens an einer ersten Region des Vorrichtungskörpers durch das Aktivierungs-/Deaktivierungsmittel moduliert werden kann, um ein anderes Wirkstoffabgabeprofil zu erzeugen als das nützliche Agens an einer zweiten Region des Vorrichtungskörpers,
**dadurch gekennzeichnet, dass** das nützliche Agens dafür konfiguriert ist, nach der Implantation innerhalb des Patienten durch das Aktivierungs-/Deaktivierungsmittel (200) in der Form eines chemischen Wirkstoffes moduliert zu werden,
wobei die bei Anwendung aus einer ersten Region der Vorrichtung (10) abgegebene Arzneimittelmenge durch das Aktivierungs-/Deaktivierungsmittel (200) moduliert werden kann, ohne das aus einer zweiten Region der Vorrichtung (10) abgegebene nützliche Agens zu modulieren.

2. Vorrichtung gemäß Anspruch 1, wobei das nützliche Agens im Wesentlichen gleichförmig in dem Vorrichtungskörper verteilt ist.

3. Vorrichtung gemäß Anspruch 1, die eine Sperrschicht umfasst, die dafür konfiguriert ist, dass durch das Aktivierungs-/Deaktivierungsmittel (200) auf sie eingewirkt wird, um das nützliches Agens in der ersten oder der zweiten Region freizusetzen oder zurückzuhalten.

4. Vorrichtung gemäß Anspruch 1, wobei das nützliche Agens in einer Matrix oder einem Bindemittel enthalten ist, die dafür konfiguriert sind, dass durch das Aktivierungs-/Deaktivierungsmittel (200) auf sie eingewirkt wird, um das nützliche Agens in der ersten oder der zweiten Region freizusetzen oder zurückzuhalten.

5. Vorrichtung gemäß Anspruch 1, wobei der Vorrichtungskörper eine zylindrische, aufweitbare medizinische Vorrichtung (10) ist.

6. Vorrichtung gemäß Anspruch 5, wobei die Vorrichtung ein Stent ist.

7. Vorrichtung gemäß Anspruch 5, wobei das nützliche Agens in mehreren Aussparungen (20) in der medizinischen Vorrichtung (10) enthalten ist.

8. Vorrichtung gemäß Anspruch 5, wobei das nützliche Agens in mehreren Durchgangslöchern (20) in der medizinischen Vorrichtung (10) enthalten ist.

9. Vorrichtung gemäß Anspruch 1, wobei das nützliche Agens dafür ausgewählt ist, eine Gefäßerkrankung zu behandeln.

10. Vorrichtung gemäß Anspruch 1, wobei das nützliche Agens in mehreren Öffnungen (20) in dem medizinischen Vorrichtungskörper (10) enthalten ist.

11. Stent gemäß Anspruch 1, der ein aufweitbarer implantierbarer Stent (10) ist, wobei das nützliche Agens an dem Stent (10) angebracht ist, wobei das nützliche Agens ein anfängliches Wirkstoff-Freisetzungsprofil hat, wobei der Stent ferner das Aktivierungs-/Deaktivierungsmittel (200) umfasst, um ein Abgabesystem für das nützliche Agens zu bilden,
wobei das Freisetzungsprofil des nützliches Agens nach der Implantation des Stents (10) innerhalb des Patienten durch das Aktivierungs-/Deaktivierungsmittel (200) moduliert werden kann, um ein Wirkstoff-Freisetzungsprofil zu erzeugen, das sich von dem anfänglichen Wirkstoff-Freisetzungsprofil unterscheidet.

12. System gemäß Anspruch 11, wobei das nützliche Agens im Wesentlichen gleichförmig in dem Vorrichtungskörper (10) verteilt ist.

13. System gemäß Anspruch 11, das eine Sperrschicht umfasst, die dafür konfiguriert ist, dass durch das Aktivierungs-/Deaktivierungsmittel (200) auf sie eingewirkt wird, um das abweichende Wirkstoff-Freisetzungsprofil zu erzeugen.

14. System gemäß Anspruch 11, wobei das nützliche Agens in einer Matrix oder einem Bindemittel enthalten ist, die dafür konfiguriert sind, dass durch das Aktivierungs-/Deaktivierungsmittel (200) auf sie eingewirkt wird, um das nützliche Agens in der ersten oder der zweiten Region freizusetzen oder zurückzuhalten.

15. System gemäß Anspruch 11, wobei das nützliche Agens in mehreren Aussparungen (20) in dem Stent (10) enthalten ist.

16. System gemäß Anspruch 11, wobei das nützliche Agens in mehreren Durchgangslöchern (20) in dem Stent (10) enthalten ist.

17. System gemäß Anspruch 11, wobei das nützliche Agens dafür ausgewählt ist, eine Gefäßerkrankung zu behandeln.

18. System gemäß Anspruch 11, wobei das nützliche Agens für im Wesentlichen keine Freisetzung bis zur Modulation durch das Aktivierungs-/Deaktivierungsmittel (200) konfiguriert ist.

19. System gemäß Anspruch 11, das ferner ein zusätzliches nützliches Agens umfasst, das an dem Stent (10) angebracht und dafür konfiguriert ist, ohne Aktivierung freigesetzt zu werden.

## Revendications

1. Stent (10) comprenant :
un corps de dispositif incluant un agent bénéfique comprenant un médicament agencé pour être libéré depuis le corps de dispositif vers un site d'implantation au sein d'un patient, l'agent bénéfique étant configuré pour être sélectivement modulé, après implantation au sein du patient, par un moyen d'activation/désactivation (200),
l'agent bénéfique, au niveau d'une première région du corps de dispositif, pouvant être modulé par le moyen d'activation/désactivation pour créer un profil de libération de l'agent différent de celui qu'a l'agent bénéfique au niveau d'une seconde région du corps de dispositif,
**caractérisé en ce que**
l'agent bénéfique est configuré pour être modulé, après implantation au sein du patient, par le moyen d'activation/désactivation (200) sous la forme d'un agent chimique, et
la quantité de médicament libéré, en service, depuis une première région du dispositif (10) peut être modulée par le moyen d'activation/désactivation (200) sans modulation de la quantité d'agent bénéfique libéré depuis une seconde région du dispositif (10).

2. Dispositif selon la revendication 1, dans lequel l'agent bénéfique est distribué de façon sensiblement uniforme dans le corps de dispositif.

3. Dispositif selon la revendication 1, comprenant une couche formant barrière, configurée pour être réceptive à l'action du moyen d'activation/désactivation (200) pour libérer ou retenir l'agent bénéfique depuis/dans la première ou la seconde région.

4. Dispositif selon la revendication 1, dans lequel l'agent bénéfique est contenu dans une matrice ou un liant configuré pour être réceptif à l'action du moyen d'activation/désactivation (200) pour libérer ou retenir l'agent bénéfique depuis/dans la première ou la seconde région.

5. Dispositif selon la revendication 1, dans lequel le corps de dispositif est un dispositif médical, cylindrique, dilatable (10).

6. Dispositif selon la revendication 5, ledit dispositif étant un stent.

7. Dispositif selon la revendication 5, dans lequel l'agent bénéfique est contenu dans une pluralité d'évidements (20) dans le dispositif médical (10).

8. Dispositif selon la revendication 5, dans lequel l'agent bénéfique est contenu dans une pluralité de trous traversants (20) dans le dispositif médical (10).

9. Dispositif selon la revendication 1, dans lequel l'agent bénéfique est sélectionné pour traiter une maladie vasculaire.

10. Dispositif selon la revendication 1, dans lequel l'agent bénéfique est situé dans une pluralité d'ouvertures (20) dans le corps de dispositif (10).

11. Stent selon la revendication 1, qui est un stent (10) implantable, dilatable, dans lequel l'agent bénéfique est solidarisé au stent (10), l'agent bénéfique ayant un profil initial de libération de l'agent, comprenant, en outre, le moyen d'activation/désactivation (200) pour former un système de libération d'agent bénéfique, où le profil de libération de l'agent bénéfique peut être modulé par le moyen d'activation/désactivation (200) après implantation du stent (10) au sein du patient pour créer un profil de libération de l'agent qui diffère du profil initial de libération de l'agent.

12. Système selon la revendication 11, dans lequel l'agent bénéfique est distribué de façon sensiblement uniforme dans le corps de dispositif.

13. Système selon la revendication 11, comprenant une couche formant barrière, configurée pour être réceptive à l'action du moyen d'activation/désactivation (200) pour créer le profil de libération de l'agent différent.

14. Système selon la revendication 11, dans lequel l'agent bénéfique est contenu dans une matrice ou un liant configuré pour être réceptif à l'action du moyen d'activation/désactivation (200) pour libérer ou retenir l'agent bénéfique depuis/dans la première ou la seconde région.

15. Système selon la revendication 11, dans lequel l'agent bénéfique est contenu dans une pluralité d'évidements (20) dans le stent (10).

16. Système selon la revendication 11, dans lequel l'agent bénéfique est contenu dans une pluralité de trous traversants (20) dans le stent (10).

17. Système selon la revendication 11, dans lequel l'agent bénéfique est sélectionné pour traiter une maladie vasculaire.

18. Système selon la revendication 11, dans lequel l'agent bénéfique est configuré pour sensiblement ne rien libérer jusqu'à ce que survienne la modulation par le moyen d'activation/désactivation (200).

19. Système selon la revendication 11, comprenant, en outre, un agent bénéfique supplémentaire solidarisé au stent (10) et configuré pour être libéré sans activation.
